(19) Europäisches Patentamt
European Patent Office
Office européen des brevets

(11) **EP 3 185 963 B1**

(12) **EUROPÄISCHE PATENTSCHRIFT**

(45) Veröffentlichungstag und Bekanntmachung des Hinweises auf die Patenterteilung:
**27.02.2019 Patentblatt 2019/09**

(21) Anmeldenummer: **15749807.2**

(22) Anmeldetag: **12.08.2015**

(51) Int Cl.:
*A61Q 5/10* (2006.01)    *A61K 8/04* (2006.01)
*A61K 8/22* (2006.01)

(86) Internationale Anmeldenummer:
**PCT/EP2015/068561**

(87) Internationale Veröffentlichungsnummer:
**WO 2016/030189 (03.03.2016 Gazette 2016/09)**

(54) **MULTITONALE EINSCHRITT-FÄRBUNGEN MIT SCHAUMARTIGER VORBEHANDLUNGSLÖSUNG**

MULTI-TONAL ONE STEP DYEING PROCESS USING A FOAM-TYPE PRETREATMENT SOLUTION

COLORATION MULTITON EN UNE ÉTAPE UTILISANT UN AGENT DE PRÉ-TRAITEMENT DE TYPE MOUSSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.08.2014 DE 102014216941**

(43) Veröffentlichungstag der Anmeldung:
**05.07.2017 Patentblatt 2017/27**

(73) Patentinhaber: **Henkel AG & Co. KGaA**
**40589 Düsseldorf (DE)**

(72) Erfinder:
• **NEUBA, Constanze**
**41516 Grevenbroich (DE)**
• **SCHETTIGER, Norbert**
**40723 Hilden (DE)**
• **MÜLLER, Burkhard**
**40221 Düsseldorf (DE)**

(56) Entgegenhaltungen:
WO-A2-01/15662     WO-A2-03/068177
DE-A1- 2 913 755     DE-A1- 3 025 992
DE-U1- 20 100 140

**Beschreibung**

Gegenstand der vorliegenden Anmeldung ist ein Verfahren zur Behandlung keratinischer Fasern, welches es erlaubt, in einem Färbeschritt das Haar zu colorieren und gleichzeitig eine multitonale Färbung mit helleren ("highlights") oder dunkleren ("lowlights") Partien (Strähnchen) zu erzeugen. Hierzu wird eine Farbstoffvorbehandlungslösung mit Oxidationsfarbstoffvor-produkten in Kombination mit einem oxidativem Färbemittel verwendet.

Im Laufe der Zeit und insbesondere unter Einwirkung von äußeren Einflüssen wie Licht oder atmosphärischen Schadstoffen verliert oder verändert das Haar seine natürliche Farbe und seinen Glanz bzw. Schimmer. Aus diesem Grund finden Haarfärbemittel breite Anwendung, welche entweder im Friseurbereich oder durch die Heimanwendung genutzt werden.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, welche unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, welche als färbende Komponente sogenannte direktziehende Farbstoffe ("Direktzieher") enthalten.

Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Sollen Substrate aufgehellt oder gar gebleicht werden, werden die das Substrat färbenden Farbstoffe meist oxidativ unter Einsatz von entsprechenden Oxidationsmitteln, wie Wasserstoffperoxid, entfärbt.

Bei der Haarfärbung - insbesondere bei der Haarfärbung durch die Heimanwendung - tritt das Problem auf, dass natürliche Farbnuancierungen vollständig überdeckt werden, so dass multitonale Färbungen schwer zu realisieren sind.

Um dem Haar ein natürlicheres Aussehen zu verleihen, ist es im Stand der Technik bekannt, gefärbte Haare durch die gezielte Anwendung von Oxidationsmitteln partiell zu entfärben. Die Haarpartien ("Strähnchen"), auf welche die Oxidationsmittel aufgetragen werden, bleichen dabei mindestens anteilsweise aus, woraus eine multitonale Haarfarbe resultiert. Die Applikation des Oxidationsmittels erfolgt dabei mit einer Bürste oder einem Pinsel, wobei die nicht zu behandelnden Haare gegebenenfalls mit Aluminiumfolie oder einer sogenannten "Strähnchenhaube" vor der Entfärbung geschützt werden.

Diese Applikationsart löst zwar das Problem der möglichst natürlichen Färbung von Haaren, lässt aber nur das Setzen von "highlights" zu. Für "lowlights", d. h. dunklere Partien, müsste nachgefärbt werden. In den zuvor beschriebenen Fällen wäre daher ein zeitaufwendiger zweiter Entfärbe- bzw. Färbeschritt erforderlich, welcher sich an die erste Färbung anschließt. Insbesondere bei der Heimanwendung müsste daher zunächst das gesamte Haar coloriert werden, bevor die Anwenderin "highlights" oder "lowlights" setzen kann. Von vielen Verbraucherinnen wird dies als zu zeitaufwendig und auch als frustrierend empfunden, da der wesentliche farbverändernde Schritt anfangs erfolgt und in einem zweiten Schritt lediglich "korrigiert" wird.

Das deutsche Gebrauchsmuster DE 201 000 140 U1 offenbart wäßrige Haarfärbemittelzusammensetzung auf Basis eines Oxidationsfarbstoffvorprodukt-Systems, enthaltend mindestens ein Tetraaminopyrimidin und/oder Triaminohydroxypyrimidin bzw. deren wasserlösliche Salze; mindestens eine Kupplersubstanz, ausgewählt aus Resorcin, 2-Methylresorcin und/oder 4-Chlorresorcin; mindestens einen direktziehenden kationischen Farbstoff und/oder einen Nitroaminobenzolfarbstoff, wobei die Zusammensetzung kein Reduktionsmittel enthält.

In der DE 29 13 755 A1 werden Verfahren zum Färben von Keratinfasern in zwei Stufen durch Abänderung des pH-Wertes offenbart.

Auch die WO 03/068177 A2 offenbart ein zweistufiges Verfahren zum Färben von Keratinfasern. Hier wird nach dem Einwirken von Farbstoffvorprodukten mit einem Entwickler nachbehandelt.

In der DE 30 25 992 werden Färbezusammensetzungen auf Basis von Paraphenylendiamin und Orthoaminophenol offenbart.

Die WO 01/15662 A1 offenbart ein Verfahren zum Färben keratinischer Fasern, bei dem in einem ersten Schritt ein Vorbehandlungsmittel auf die Fasern aufgetragen wird, das mindestens einen direktziehenden Farbstoff enthält, und in einem zweiten Schritt die Fasern einer herkömmlichen Färbung mit mindestens einem synthetischen Farbstoff(vorprodukt) unterzogen werden.

Der vorliegenden Erfindung lag die Aufgabe zugrunde, ein Verfahren bereitzustellen, welches multitonale Färbungen in einem Färbeschritt ermöglicht. Dabei sollte die Färbung des Haares mit der Erzeugung von "highlights" oder "lowlights" einhergehen, damit nach dem Ausspülen des Färbemittels direkt ein Ergebnis sichtbar ist. Weiterhin sollte die Erzeugung der "highlights" oder "lowlights" ohne Verwischen des Strähnchenergebnisses ermöglicht werden.

Es wurde nun gefunden, dass eine partielle Vorbehandlung von Faserarealen bzw. Strähnchen dazu führt, dass diese Areal bzw. Strähnchen später intensiver oder weniger intensiv angefärbt werden. Durch die Vorpenetration bzw. Vorbehandlung einzelner Faserareale bzw. Strähnchen färbt das unmittelbar darauffolgend angewendete Färbemittel das Haar multitonal und es wird direkt nach dem Färbeschritt ein natürliches Färbeergebnis mit "highlights" oder "lowlights" erhalten. Die Trocknung der Vorpenetrationslösung verhindert ein Verwischen bzw. Verlaufen dieser Lösung auf andere Haarpartien und somit auch ein Verwischen des Strähnchenergebnisses.

Ein Gegenstand der vorliegenden Erfindung ist daher ein Verfahren zum oxidativen Färben von keratinischen Fasern, wobei das Verfahren die folgenden Verfahrensschritte in der angegebenen Reihenfolge umfasst:

a) Applikation eines kosmetischen Mittels (M1) auf die keratinischen Fasern, wobei das kosmetische Mittel (M1) nur auf einzelne Strähnchen appliziert wird,

b) Einwirkenlassen des Mittels (M1) für einen Zeitraum von 30 Sekunden bis 35 Minuten bei einer Temperatur von 30 °C bis 120 °C auf den keratinischen Fasern,

c) Applikation eines kosmetischen Mittels (M2) auf die mit dem kosmetischen Mittel (M1) beaufschlagten keratinischen Fasern,

d) Einwirkenlassen der kosmetischen Mittel (M1) und (M2) für einen Zeitraum von 1 bis 70 Minuten auf den keratinischen Fasern,

e) Ausspülen der kosmetischen Mittel (M1) und (M2),

dadurch gekennzeichnet, dass

- das kosmetische Mittel (M1)
  mindestens ein Oxidationsfarbstoffvorprodukt vom Entwicklertyp enthält (M1-1),
  mindestens ein Oxidationsfarbstoffvorprodukt vom Kupplertyp enthält (M1-2),
  mindestens einen direktziehenden Farbstoff enthält (M1-3), und
  mindestens ein Tensid in einer Gesamtmenge von 4,0 bis 12 Gew.-%, bezogen auf das Gesamtgewicht des kosmetischen Mittels (M1), enthält (M1-4),
  einen pH-Wert von 9,2 bis 10,5 aufweist,
  0 Gew.-%, bezogen auf das Gesamtgewicht des kosmetischen Mittels (M1), Oxidationsmittel enthält,
- das kosmetische Mittel (M2)
  mindestens ein Oxidationsfarbstoffvorprodukt enthält (M2-1), und
  mindestens ein Oxidationsmittel enthält (M2-2),
- das kosmetischen Mittel (M1) und das kosmetischen Mittel (M2) identische pH-Werte aufweisen.

Unter dem Begriff "keratinische Fasern oder auch Keratinfasern" sind erfindungsgemäß Pelze, Wolle, Federn sowie menschliche Haare zu verstehen. Im Rahmen der vorliegenden Erfindung ist es besonders bevorzugt, wenn das erfindungsgemäße Verfahren zur Färbung von menschlichen Haaren verwendet wird.

Erfindungsgemäß bevorzugt werden die Verfahrensschritte a) bis e) in der zuvor angeführten Reihenfolge mit einem Zeitabstand zwischen den einzelnen Verfahrensschritten von jeweils 0 bis 60 Minuten, vorzugsweise von jeweils 0 bis 40 Minuten, insbesondere von jeweils 0 bis 30 Minuten, durchgeführt.

Im ersten Verfahrensschritt (Verfahrensschritt a)) des erfindungsgemäßen Verfahrens wird ein kosmetisches Mittel (M1) auf die Fasern appliziert. Dieses kosmetische Mittel (M1), welches nachfolgend auch als Vorbehandlungsmittel oder als Vorpenetrationsmittel bezeichnet wird, wird über einen Zeitraum von 30 Sekunden bis 35 Minuten bei einer Temperatur von 25 °C bis 120 °C auf den keratinischen Fasern belassen (Verfahrensschritt b) des erfindungsgemäßen Verfahrens).

Die Vorbehandlung von keratinischen Fasern mit dem kosmetischen Mittel (M1) führt dazu, dass an diesen Stellen bereits Inhaltsstoffe des Vorbehandlungsmittels (M1) an den keratinischen Fasern haften bzw. in die keratinischen Fasern eingedrungen sind, so dass das Färbeergebnis bei anschließendem Auftragen des kosmetischen Mittels (M2) an diesen Stellen verstärkt bzw. aufgehellt wird. Auf diese Weise kann in einem Färbeschritt das Haar coloriert und gleichzeitig eine multitonale Färbung mit helleren ("highlights") oder dunkleren ("lowlights") Partien (Strähnchen) erzeugt werden. Aufgrund der höheren Temperaturen während der Einwirkzeit des Vorbehandlungsmittels (M1), welche beispielsweise mittels eines Heißluftföns oder einer Trockenhaube erreicht werden können, wird eine vorzugsweise vollständige Trocknung des Vorbehandlungsmittels (M1) auf den keratinischen Fasern erreicht. Diese Trocknung verhindert zum einen ein Verwischen bzw. Verlaufen des Vorbehandlungsmittels (M1) bei der nachfolgenden Applikation des kosmetischen Mittels (M2) und führt zum anderen zu einer ausreichenden Trennung der mit dem Vorbehandlungsmittel (M1) behandelten Strähnchen. Auf diese Weise wird ein Verwischen des Strähnchenergebnisses bei Durchführung des erfindungsgemäßen Verfahrens vermieden.

Um multitonale Färbungen zu erzeugen, wird das kosmetische Mittel (M1) nicht gleichmäßig auf die keratinischen Fasern appliziert. Vielmehr wird das kosmetische Mittel (M1) nur auf einzelne Strähnchen appliziert.

Unter dem Begriff "Strähnchen" wird erfindungsgemäß ein von der Gesamtheit an keratinischen Fasern abgetrennter Teil verstanden, welcher aus mindestens 2, bevorzugt mindestens 50, insbesondere mindestens 100, keratinischen Fasern besteht.

Im Anschluss an die Einwirkzeit des Vorbehandlungsmittels werden die keratinischen Fasern nicht ausgespült oder abfrottiert. Vielmehr wird in Verfahrensschritt c) des erfindungsgemäßen Verfahrens ein kosmetisches Mittel (M2) auf die noch mit dem kosmetischen Mittel (M1) beaufschlagten keratinischen Fasern appliziert. Die durch Applikation des kosmetischen Mittels (M2) auf den keratinischen Fasern entstandene Mischung aus den kosmetischen Mitteln (M1) und (M2) wird in Verfahrensschritt d) des erfindungsgemäßen Verfahrens für einen Zeitraum von 1 bis 70 Minuten Einwirken gelassen.

Erfindungsgemäß sind jedoch eher kürzere Einwirkzeiten der kosmetischen Mittel (M1) und (M2) in Verfahrensschritt d) bevorzugt. Besonders bevorzugte erfindungsgemäße Verfahren sind dadurch gekennzeichnet, dass die kosmetischen Mittel (M1) und (M2) in Verfahrensschritt d) für einen Zeitraum von 1 bis 60 Minuten, vorzugsweise von 5 bis 50 Minuten, insbesondere von 10 bis 45 Minuten, Einwirken gelassen werden.

Da das kosmetische Mittel (M1) in Schritt b) des erfindungsgemäßen Verfahrens bereits eine gewisse Zeit auf den keratinischen Fasern belassen wurde, haben diese keratinischen Fasern zu den Inhaltsstoffen des kosmetischen Mittels (M1) einen längeren Kontakt als zu denen des kosmetischen Mittels (M2). Wenn das kosmetische Mittel (M1) nur auf einzelne Strähnchen bzw. in einzelnen Bereichen abgewendet wurde, konnten die Inhaltsstoffe des kosmetischen Mittels (M1) in diesen Bereichen intensiver einwirken und somit die Wirkung der Inhaltsstoffe des kosmetischen Mittels (M2) in diesen Bereichen verstärken oder abschwächen, wodurch eine dunklere oder hellere Färbung dieser Bereiche erzielt wird.

Nach dem Ausspülen der kosmetischen Mittel (M1) und (M2) in Schritt e) des erfindungsgemäßen Verfahrens wird, ohne einen weiteren Schritt durchführen zu müssen, unmittelbar ein multitonales Farbergebnis erhalten.

Bei dem kosmetischen Mittel (M1) bzw. dem Vorbehandlungsmittel handelt es sich um ein oxidatives Haarfärbemittel, welches mindestens ein Oxidationsfarbstoffvorprodukt vom Entwicklertyp (M1-1) enthält. Erfindungsgemäß bevorzugt ist das mindestens eine Oxidationsfarbstoffvorprodukt vom Entwicklertyp (M1-1) ausgewählt aus der Gruppe von 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on, p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol sowie deren physiologisch verträglichen Salzen und deren Mischungen.

Um natürliche Färbungen zu erhalten, müssen üblicherweise mehrere Oxidationsfarbstoffvorprodukte vom Entwicklertyp eingesetzt werden. Bevorzugte kosmetische Mittel (M1) sind daher dadurch gekennzeichnet, dass das mindestens eine Oxidationsfarbstoffvorprodukt vom Entwicklertyp (M1-1) ausgewählt ist aus mindestens einer der folgenden Kombinationen: p-Toluylendiamin/2-(2-Hydroxyethyl)-p-phenylendiamin; p-Toluylendiamin/2-Methoxymethyl-p-phenylendiamin; p-Toluylendiamin/N,N-Bis-(2-hydroxyethyl)-p-phenylendiamin; p-Toluylendiamin/2-Methoxymethyl-p-phenylendiamin; p-Toluylendiamin/N-(4-Amino-3-methylphenyl)-N-[3-(1H-imidazol-1-yl)propyl]amin; p-Toluylendiamin/Bis-(2-hydroxy-5-aminophenyl)methan; p-Toluylendiamin/4-Amino-3-methylphenol; p-Toluylendiamin/4,5-Diamino-1-(2-hydroxyethyl)pyrazol; p-Toluylendiamin/2,4,5,6-Tetraaminopyrimidin; 2-(2-Hydroxyethyl)-p-phenylendiamin /2-Methoxymethyl-p-phenylendiamin; 2-(2-Hydroxyethyl)-p-phenylendiamin /N,N-Bis-(2-hydroxyethyl)-p-phenylendiamin; 2-(2-Hydroxyethyl)-p-phenylendiamin /2-Methoxymethyl-p-phenylendiamin; 2-(2-Hydroxyethyl)-p-phenylendiamin /N-(4-Amino-3-methylphenyl)-N-[3-(1H-imidazol-1-yl)propyl]amin; 2-(2-Hydroxyethyl)-p-phenylendiamin /Bis-(2-hydroxy-5-aminophenyl)methan; 2-(2-Hydroxyethyl)-p-phenylendiamin/4-Amino-3-methylphenol; 2-(2-Hydroxyethyl)-p-phenylendiamin /4,5-Diamino-1-(2-hydroxyethyl)pyrazol; 2-(2-Hydroxyethyl)-p-phenylendiamin /2,4,5,6-Tetraaminopyrimidin; 2-Methoxymethyl-p-phenylendiamin/2-(2-Hydroxyethyl)-p-phenylendiamin; 2-Methoxymethyl-p-phenylendiamin /2-Methoxymethyl-p-phenylendiamin; 2-Methoxymethyl-p-phenylendiamin /N,N-Bis-(2-hydroxyethyl)-p-phenylendiamin; 2-Methoxymethyl-p-phenylendiamin /2-Methoxymethyl-p-phenylendiamin; 2-Methoxymethyl-p-phenylendiamin /N-(4-Amino-3-methylphenyl)-N-[3-(1H-imidazol-1-yl)-propyl]amin; 2-Methoxymethyl-p-phenylendiamin /Bis-(2-hydroxy-5-aminophenyl)methan; 2-Methoxymethyl-p-phenylendiamin/4-Amino-3-methylphenol; 2-Methoxymethyl-p-phenylendiamin /4,5-Diamino-1-(2-hydroxyethyl)pyrazol; 2-Methoxymethyl-p-phenylendiamin /2,4,5,6-Tetraaminopyrimidin und/oder 4-und Amino-3-methylphenol/4,5-Diamino-1-(2-hydroxyethyl)pyrazol und/oder deren physiologisch verträgliche Salze.

Gemäß einer besonders bevorzugten Ausführungsform des ersten Erfindungsgegenstands ist das mindestens eine Oxidationsfarbstoffvorprodukt vom Entwicklertyp (M1-1) ausgewählt aus der Gruppe von p-Toluylendiamin, 4-Amino-3-methylphenol, 4,5-Diamino-1-(2-hydroxyethyl)pyrazol, 2,4,5,6-Tetraaminopyrimidin und/oder deren physiologisch verträglichen Salzen sowie deren Mischungen. Es hat sich gezeigt, dass der Einsatz dieser speziellen Oxidationsfarbstoffvorprodukte vom Entwicklertyp (M1-1) in den im Rahmen des erfindungsgemäßen Verfahrens eingesetzten Vorbehandlungsmittel (M1) zu besonders lebendigen, wasch-, reib-, schweiß- und UV-echten multitonalen Färbungen führt.

Besonders ansprechend aussehende multitonale Färbungen werden erhalten, wenn das kosmetische Mittel (M1) das mindestens eine Oxidationsfarbstoffvorprodukt vom Entwicklertyp (M1-1) in einer Gesamtmenge von 0,0025 bis 10,0 Gew.-%, vorzugsweise von 0,004 bis 8,0 Gew.-%, bevorzugt 0,005 bis 5,0 Gew.-%, insbesondere 0,01 bis 3,5 Gew.-%, bezogen auf das Gesamtgewicht des kosmetischen Mittels (M1), enthält. Die vorstehend genannten Mengen der Entwicklerkomponente (M1-1) führen zu multitonalen Färbungen, welche besonders intensive und leuchtende Farben sowie eine hohe Beständigkeit gegen Umwelteinflüsse, wie Haarwäschen, UV-Licht, Schweiß und Reibung, aufweisen.

Das Vorbehandlungsmittel (M1) enthält als weiteren Bestandteil mindestens ein Oxidationsfarbstoffvorprodukt vom Kupplertyp (M1-2). Oxidationsfarbstoffvorprodukte vom Kupplertyp bilden im Rahmen der oxidativen Färbung allein keine signifikante Färbung aus, sondern benötigen für eine ausreichende Färbung die Gegenwart von Oxidationsfarbstoffvorprodukten vom Entwicklertyp. Oxidationsfarbstoffvorprodukte vom Kupplertyp im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus.

Im Rahmen der vorliegenden Erfindung ist es bevorzugt, wenn das mindestens eine Oxidationsfarbstoffvorprodukt vom Kupplertyp (M1-2) ausgewählt ist aus der Gruppe von m-Aminophenol und dessen Derivaten, o-Aminophenol und dessen Derivaten, m-Diaminobenzol und dessen Derivaten, o-Diaminobenzol und dessen Derivaten, Di- und Trihydroxybenzolderivaten, Pyridinderivaten, Naphthalinderivaten, Morpholinderivaten, Chinoxalinderivaten, Pyrazolderivaten, Indolderivaten, Pyrimidinderivaten, Methylendioxybenzolderivaten und/oder deren physiologisch verträglichen Salzen sowie deren Mischungen.

Bevorzugte erfindungsgemäße Verfahren sind dadurch gekennzeichnet, dass das mindestens eine Oxidationsfarbstoffvorprodukt vom Kupplertyp (M1-2) ausgewählt ist aus der Gruppe von Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin, 1-Naphthol und/oder deren physiologisch verträglichen Salzen sowie deren Mischungen. Die vorstehend genannten Kupplerkomponenten (M1-2) führen in Kombination mit der mindestens einen Entwicklerkomponente (M1-1) in den Vorbehandlungsmitteln (M1) zu besonders intensiven und beständigen multitonalen Färbeergebnissen.

Erfindungsgemäß bevorzugt enthält das kosmetische Mittel (M1) das mindestens eine Oxidationsfarbstoffvorprodukt vom Kupplertyp (M1-2) in einer Gesamtmenge von 0,001 bis 6,0 Gew.-%, vorzugsweise von 0,001 bis 5,5 Gew.-%, bevorzugt von 0,002 bis 4,5 Gew.-%, insbesondere von 0,005 bis 2,5 Gew.-%, bezogen auf das Gesamtgewicht des kosmetischen Mittels (M1). Die vorstehend genannten Mengen der Kupplerkomponente (M1-2) in den im Rahmen des erfindungsgemäßen Verfahrens eingesetzten Vorbehandlungsmittel (M1) führen zu besonders lebendigen, wasch-, reib-, schweiß- und UV-echten multitonalen Färbungen.

Um eine ausgewogene und subtile Nuancenausbildung zu gewährleisten, enthalten die im Rahmen des erfindungsgemäßen Verfahrens eingesetzten Vorbehandlungsmittel (M1) mindestens einen direktziehenden Farbstoff (M1-3). Bei direktziehenden Farbstoffen handelt es sich um Farbstoffe, welche direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Gemäß einer bevorzugten Ausführungsform des ersten Erfindungsgegenstands ist der mindestens eine direktziehende Farbstoff (M1-3) ausgewählt aus der Gruppe von anionischen direktziehenden Farbstoffen, kationischen direktziehenden Farbstoffen, nichtionischen direktziehenden Farbstoffen sowie deren Mischungen.

In diesem Zusammenhang kann es erfindungsgemäß vorgesehen sein, dass der anionische direktziehende Farbstoff ausgewählt ist aus der Gruppe von Acid Yellow 1, Acid Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Tetrabromphenolblau und/oder deren physiologisch verträglichen Salzen.

Im Rahmen dieser Ausführungsform kann es weiterhin vorgesehen sein, dass der kationische direktziehende Farbstoff ausgewählt ist aus der Gruppe von Basic Blue 7, Basic Blue 26, Basic Violet 2 und Basic Violet 14, Basic Yellow 57, Basic Red 76, Basic Blue 99, Basic Brown 16 und Basic Brown 17 und HC Blue 16, sowie Basic Yellow 87, Basic Orange 31 und Basic Red 51 und/oder deren physiologisch verträglichen Salzen.

Darüber hinaus kann es im Rahmen dieser Ausführungsform auch vorgesehen sein, dass der nichtionische direktziehende Farbstoff ausgewählt ist aus der Gruppe von HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure, 2-Amino-6-chloro-4-nitrophenol, 4-Ethylamino-3-nitrobenzoesäure und 2-Chlor-6-ethylamino-4-nitrophenol und/oder deren physiologisch verträglichen Salzen, bevorzugt 2-Amino-6-chloro-4-nitrophenol und/oder 4-Amino-3-nitrophenol und/oder deren physiologisch verträglichen Salzen.

Im Rahmen des erfindungsgemäßen Verfahrens besonders bevorzugt eingesetzte Vorbehandlungsmittel (M1) enthalten mindestens eine direktziehende Farbstoff (M1-3), welcher ausgewählt ist aus der Gruppe von 2-Amino-6-chloro-4-nitrophenol, HC Blue 12, HC Yellow 2, HC Violet 14D und/oder deren physiologisch verträglichen Salzen sowie deren Mischungen. Bei Einsatz dieser speziellen direktziehenden Farbstoffe wird eine besonders ausgewogene und subtile Nuancenbildung während des erfindungsgemäßen Verfahrens bzw. bei der multitonalen Färbung erreicht.

Erfindungsgemäß bevorzugt enthält das kosmetische Mittel (M1) den mindestens einen direktziehenden Farbstoff (M1-3) in einer Gesamtmenge von 0,0001 bis 6,0 Gew.-%, vorzugsweise von 0,0005 bis 5,5 Gew.-%, bevorzugt von 0,0008 bis 5,0 Gew.-%, weiter bevorzugt von 0,005 bis 4,5 Gew.-%, insbesondere von 0,001 bis 3,5 Gew.-%, bezogen auf das Gesamtgewicht des kosmetischen Mittels (M1). Die zuvor genannten Mengen der direktziehenden Farbstoffe führen zu besonders ausgewogenen Nuancen der multitonalen Färbung gemäß dem erfindungsgemäßen Verfahren.

Das im Rahmen des erfindungsgemäßen Verfahrens eingesetzte Vorbehandlungsmittel (M1) enthält weiterhin mindestens ein Tensid (M1-4). Tenside im Sinne der vorliegenden Erfindung sind amphiphile (bifunktionelle) Verbindungen, welche aus mindestens einem hydrophoben und mindestens einem hydrophilen Molekülteil bestehen. Eine Basiseigenschaft von Tensiden und Emulgatoren ist die orientierte Absorption an Grenzflächen sowie die Aggregation zu Mizellen und die Ausbildung von lyotrophen Phasen.

Gemäß einer bevorzugten Ausführungsform des ersten Erfindungsgegenstands ist das mindestens eine Tensid (M1-4) ausgewählt aus der Gruppe von nichtionischen Tensiden, anionischen Tensiden, amphoteren Tensiden, zwitterionischen Tensiden, kationischen Tensiden sowie deren Mischungen.

In diesem Zusammenhang kann es erfindungsgemäß vorgesehen sein, dass das nichtionische Tensid ausgewählt ist aus der Gruppe von ethoxylierten Alkoholen und Carbonsäuren mit 8 bis 13 Kohlenstoffatomen und 2 bis 30 Ethylenoxideinheiten, Anlagerungsprodukten von 5 bis 60 Mol Ethylenoxid an Rizinusöl und gehärtetes Rizinusöl, Alkylpolyglucosiden der Formel $R^1O\text{-}[G]_p$, in welcher $R^1$ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, Monoethanolamiden von Carbonsäuren mit 8 bis 30 Kohlenstoffatomen sowie deren Mischungen.

Im Rahmen dieser Ausführungsform kann es weiterhin vorgesehen sein, dass das anionische Tensid ausgewählt ist aus der Gruppe von Alkylsulfaten und Alkylpolyglycolethersulfaten der Formel $R^2\text{-}O(CH_2\text{-}CH_2O)_x\text{-}OSO_3H$, in welcher $R^2$ eine lineare Alkylgruppe mit 8 bis 30 Kohlenstoffatomen und x = 0 oder 1 bis 12 ist, Salzen linearer und verzweigter Carbonsäuren mit 8 bis 30 Kohlenstoffatomen, Ethercarbonsäuren der Formel $R^3\text{-}O\text{-}(CH_2\text{-}CH_2O)_x\text{-}CH_2\text{-}CO\text{-}OH$, in welcher $R^3$ eine lineare Alkylgruppe mit 8 bis 30 C-Atomen und x = 0 oder 1 bis 16 ist, sowie deren Mischungen.

Darüber hinaus kann es im Rahmen dieser Ausführungsform auch vorgesehen sein, dass das amphotere Tensid ausgewählt ist aus der Gruppe von Amphoacetaten mit Carbonsäureresten mit 8 bis 30 Kohlenstoffatomen, N-Alkylglycinen, N-Alkylpropionsäuren, N-Alkylamidobuttersäuren, N-Alkyliminodipropionsäuren, N-Hydroxyethyl-N-alkylamidopropylglycinen, N-Alkyltaurinen, N-Alkylsarcosinen, 2-Alkylaminopropionsäuren, Alkylaminoessigsäuren sowie deren Mischungen.

Im Rahmen dieser Ausführungsform kann es zudem vorgesehen sein, dass das zwitterionische Tensid ausgewählt ist aus der Gruppe von Betainen, N-Alkyl-N,N-diemthylammoniumglycinaten, N-Acylamidopropyl-N,N-dimethylammoniumglycinaten, 2-Alkyl-3-carboxymethyl-3-hydroxyethyl-imidazolinen sowie deren Mischungen.

Im Rahmen des erfindungsgemäßen Verfahrens besonders bevorzugt eingesetzte Vorbehandlungsmittel (M1) enthalten mindestens ein Tensid (M1-4), welches ausgewählt ist aus der Gruppe von Alkylpolyglucosiden der Formel $R^1O\text{-}[G]_p$, in welcher $R^1$ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, Monoethanolamide von Carbonsäuren mit 8 bis 30 Kohlenstoffatomen, Amphoacetaten mit Carbonsäureresten mit 8 bis 30 Kohlenstoffatomen sowie deren Mischungen. Der Einsatz der zuvor genannten Tenside führt zu einer besonders guten Schaumqualität und somit zu einer hohen Benetzung der keratinischen Fasern mit dem Vorbehandlungsmittel (M1). Weiterhin führt der Einsatz dieser Tenside zu einer guten Verteilbarkeit der Vorbehandlungsmittel (M1) in Verfahrensschritt a) und zu einer ausreichenden, insbesondere vollständigen, Trocknung der Vorbehandlungsmittel (M1) während der Einwirkzeit in Verfahrensschritt b). Durch die Trocknung des Vorbehandlungsmittels (M1) auf den keratinischen Fasern wird ein Verwischen bei Auftragen des kosmetischen Mittels (M2) in Verfahrensschritt c) und somit auch ein Verwischen des multitonalen Färbeergebnisses vermieden.

Erfindungsgemäß bevorzugt enthält das kosmetische Mittel (M1) das mindestens eine Tensid (M1-4) in einer Gesamtmenge von 4,0 bis 12 Gew.-%, bezogen auf das Gesamtgewicht des kosmetischen Mittels (M1). Die zuvor genannten Mengen der Tenside gewährleisten ein gute Schaumqualität sowie eine ausreichend schnelle Trocknung des Vorbehandlungsmittels (M1) in Verfahrensschritt b) des erfindungsgemäßen Verfahrens.

Um das Vorpenetrationsmittel (M1) sauber und lokal begrenzt applizieren zu können, hat sich eine bestimmte Viskosität des Mittels als vorteilhaft erwiesen. Dazu ist es vorteilhaft, wenn das Mittel nicht als Paste, zähe Creme oder eingedicktes Gel vorliegt, sondern über eine ausreichende Fließfähigkeit verfügt. Weiterhin muss das kosmetische Mittel (M1) zwar über rheologische Eigenschaften verfügen, welche ein Auftragen auf die zu färbenden Fasern erlauben, aber gleichzeitig auch ein Verlaufen oder Ausfließen des kosmetischen Mittels (M1) von den keratinischen Fasern, insbesondere unter Einwirkung von Wärme, während der Einwirkzeit in Verfahrensschritt b) verhindern. Erfindungsgemäß bevorzugt verwendete kosmetische Mittel (M1) weisen daher eine dynamische Viskosität von 10 bis 300 mPa*s, vorzugsweise von 20 bis 200 mPa*s, bevorzugt von 30 bis 100 mPa*s, weiter bevorzugt von 40 bis 90 mPa*s, insbesondere von 50 bis 80 mPa*s, jeweils gemessen mit Brookfield RDV II+, Spindel Nr. 1, 100 rpm, 20 °C, auf.

Im Rahmen einer besonders bevorzugten Ausführungsform des ersten Erfindungsgegenstands liegt das kosmetische Mittel (M1) als Schaum vor und weist eine Flüssigkeitsabscheidung nach 10 Minuten von 0,0 bis 60 %, vorzugsweise von 0,0 bis 50 %, insbesondere von 0,0 bis 40 %, auf. Vorbehandlungsmittel (M1), welche als Schaum vorliegen und die zuvor angeführte Flüssigkeitsabscheidung aufweisen erlauben zum einen eine vollständig Benetzung der mit diesem Mittel behandelten keratinischen Fasern. Zum anderen gewährleistet diese Konsistenz eine ausreichende, insbesondere vollständige, Trocknung des schaumförmigen Vorbehandlungsmittels (M1) mittels Wärme in Verfahrensschritt b). Diese bevorzugt vollständige Trocknung des Vorbehandlungsmittels (M1) verhindert ein Verwischen dieses Mittels bei Auftragung des kosmetischen Mittels (M2) und somit ein Verwischen des multitonalen Färbeergebnisses.

Die Flüssigkeitsabscheidung des kosmetischen Mittels (M1) nach 10 Minuten kann beispielsweise mit folgender Messmethode bestimmt werden: Zunächst wird das Gewicht eines bestimmten Volumens, beispielsweise 80 ml, des schaumförmigen kosmetischen Mittels (M1) bestimmt (Referenzwert). Nach 10 Minuten wird die abgeschiedene Flüssigkeit dekantiert und das Gewicht dieser Flüssigkeit bestimmt. Die Flüssigkeitsabscheidung in % errechnet sich nach folgender Formel:

$$\text{Flüssigkeitsabscheidung nach 10 Minuten} = [\text{Gewicht Flüssigkeit (g) / Gewicht Mittel (M1) (g)}] *100$$

Die im Rahmen des erfindungsgemäßen Verfahrens verwendeten Vorbehandlungsmittel (M1) können als treibmittelfreies kosmetisches Mittel oder als treibmittelhaltiges kosmetisches Mittel konfektioniert sein. Treibmittelfreie kosmetische Mittel (M1) können in jedem beliebigen treibgasfreien Spraysystem, welches einen Spendebehälter und ein Sprühventil aufweist, ausgebracht werden, also z. B. in einer flexiblen Druckflasche mit Tauchrohr und Sprühventil (Squeeze Bottle) oder in einer Pumpen-Sprühflasche, deren Pumpenhebel mit dem Zeigefinger oder mit der ganzen Hand in der Art eines Abzugsbügels betätigt wird.

Sind die Vorbehandlungsmittel (M1) als treibmittelhaltige kosmetische Mittel konfektioniert, enthalten sie zusätzlich mindestens ein Treibmittel. Im Rahmen dieser Ausführungsform enthält das kosmetische Mittel (M1) mindestens ein Treibmittel, ausgewählt aus der Gruppe von $N_2O$, Dimethylether, $CO_2$, Luft, Propan, n-Butan, iso-Butan, n-Pentan, iso-Pentan, sowie deren Mischungen, bevorzugt eine Mischung aus Propan und n-Butan im Gewichtsverhältnis von 15:85.

Im Rahmen dieser Ausführungsform ist es weiterhin bevorzugt, wenn das kosmetische Mittel (M1) das mindestens eine Treibmittel in einer Gesamtmenge von 0,5 bis 16 Gew.-%, vorzugsweise von 1,0 bis 14 Gew.-%, bevorzugt von 3,0 bis 13 Gew.-%, weiter bevorzugt von 4,0 bis 12 Gew.-%, insbesondere von 5,5 bis 10 Gew.-%, bezogen auf das Gesamtgewicht des kosmetischen Mittels (M1), enthält.

Zusätzlich zu dem bzw. den Oxidationsfarbstoffvorprodukt(en) vom Entwicklertyp und Kupplertyp, dem bzw. den direktziehenden Farbstoff(en) sowie dem bzw. den Tensid(en) kann das in dem erfindungsgemäßen Verfahren eingesetzte kosmetische Mittel (M1) weitere Inhaltsstoffe enthalten.

Erfindungsgemäß bevorzugt enthält das kosmetische Mittel (M1) zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Alkalisierungsmitteln; sowie (iv) deren Mischungen.

Um die gewünschte Viskosität einzustellen, können die kosmetischen Mittel (M1) mindestens ein Verdickungsmittel und/oder mindestens einen Gelbildner enthalten.

In diesem Zusammenhang kann es daher vorgesehen sein, dass die kosmetischen Mittel (M1) mindestens ein anionisches, polymeres Verdickungsmittel enthalten. Bevorzugte anionische polymere Verdickungsmittel sind ausgewählt aus vernetzten oder unvernetzten Copolymeren, welche mindestens zwei unterschiedliche Monomere aus der Gruppe der Acrylsäure, Methacrylsäure, den $C_1$-$C_6$-Alkylestern der Acrylsäure und/oder den $C_1$-$C_6$-Alkylestern der Methacrylsäure enthalten. Besonders bevorzugte anionische Copolymere sind Copolymere aus Acrylsäure, Methacrylsäure oder deren $C_1$-$C_6$-Alkylestern, welche unter der INCI-Bezeichnung Acrylates Copolymer vertrieben werden. Insbesondere bevorzugt ist die Kombination aus Methacrylsäure und Ethylacrylat sowie gegebenenfalls vernetzenden, multifunktionalen Monomeren. Ein bevorzugtes Handelsprodukt dafür ist beispielsweise Aculyn® 33 bzw. 33A, welches von der Firma Rohm & Haas angeboten wird.

Die anionischen polymeren Verdickungsmittel können in einer Gesamtmenge von 0,1 bis 15 Gew.-%, vorzugsweise von 1 bis 10 Gew.-%, insbesondere von 1,5 bis 7,5 Gew.-%, bezogen auf das Gesamtgewicht des kosmetischen Mittels (M1), eingesetzt werden.

Die erfindungsgemäß in Verfahrensschritt b) verwendeten Vorbehandlungsmittel (M1) können ebenfalls lineare oder verzweigte, gesättigte oder ungesättigte Alkohole mit 8 bis 20 Kohlenstoffatomen enthalten. Es hat sich herausgestellt, dass die zusätzliche Anwesenheit dieser höherkettigen Alkohole das multitonale Färbeergebnis des erfindungsgemäßen Verfahrens noch weiter verbessern kann. Daher ist es bevorzugt, wenn die im erfindungsgemäßen Verfahren eingesetzten Vorbehandlungsmittel (M1) zusätzlich einen oder mehrere Alkohole aus der Gruppe von Arachylalkohol (Eisocan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), Heneicosylalkohol (Heneicosan-1-ol), Behenylalkohol (Docosan-1-ol), Erucylalkohol ((13Z)-Docos-13-en-1-ol) und Brassidylalkohol ((13E)-Docosen-1-ol) enthalten.

Besonders geeignete kosmetische Mittel (M1) enthalten einen oder mehrere höherkettige Alkohole der vorgenannten Gruppe in einer Gesamtmenge von 1,0 bis 10,0 Gew.-%, vorzugsweise von 1,4 bis 8,0 Gew.-%, bevorzugt von 1,8 bis 6,0 Gew.-%, insbesondere von 2,0 bis 4,0 Gew.-%, bezogen auf das Gesamtgewicht des kosmetischen Mittels (M1).

In einer weiteren bevorzugten Ausführungsform ist ein in dem erfindungsgemäßen Verfahren eingesetztes kosmetisches Mittel (M1) daher dadurch gekennzeichnet, dass es zusätzlich einen oder mehrere Alkohole aus der Gruppe von Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), Heneicosylalkohol (Heneicosan-1-ol), Behenylalkohol (Docosan-1-ol), Erucylalkohol ((13Z)-Docos-13-en-1-ol) und Brassidylalkohol ((13E)-Docosen-1-ol) in einer Gesamtmenge von 0,1 bis 10,0 Gew.-%, vorzugsweise von 1,4 bis 8,0 Gew.-%, bevorzugt von 1,8 bis 6,0 Gew.-%, insbesondere von 2,0 bis 4,0 Gew.-%, bezogen auf das Gesamtgewicht des kosmetischen Mittels (M1), enthält.

Die im Rahmen des erfindungsgemäßen Verfahrens verwendeten Vorbehandlungsmittel (M1) können ebenfalls mindestens ein Alkalisierungsmittel enthalten.

Erfindungsgemäß einsetzbare, organische Alkalisierungsmittel werden bevorzugt ausgewählt aus Alkanolaminen aus primären, sekundären oder tertiären Aminen mit einem $C_2$-$C_6$-Alkylgrundkörper, welcher mindestens eine Hydroxylgruppe trägt. Erfindungsgemäß ganz besonders bevorzugte Alkanolamine werden ausgewählt aus der Gruppe 2-Aminoethan-1-ol (Monoethanolamin), 2-Amino-2-methylpropan-1-ol und 2-Amino-2-methyl-propan-1,3-diol sowie deren Mischungen. Ein insbesondere bevorzugtes Alkanolamin ist Monoethanolamin. Geeignete basische Aminsäuren sind Lysin, Arginin und Ornithin. Erfindungsgemäße, anorganische Alkalisierungsmittel sind bevorzugt ausgewählt aus der Gruppe von Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat sowie deren Mischungen.

Erfindungsgemäß besonders bevorzugte Verfahren sind dadurch gekennzeichnet, dass das kosmetische Mittel (M1) ein oder mehrere Alkalisierungsmittel aus der Gruppe von Natriumhydroxid, Kaliumhydroxid, Ammoniak, Monoethanolamin und 2-Amino-2-methylpropanol in einer Gesamtmenge von 0,05 bis 8,0 Gew.-%, vorzugsweise von 0,1 bis 6,0 Gew.-%, insbesondere von 0,5 bis 5,0 Gew.-%, bezogen auf das Gesamtgewicht des kosmetischen Mittels (M1), enthält.

Die im Rahmen des erfindungsgemäßen Verfahrens verwendeten Vorbehandlungsmittel (M1) weisen in der Regel einen basischen pH-Wert, insbesondere zwischen pH 7,0 und pH 14, auf. Diese pH-Werte sind erforderlich, um eine Öffnung der äußeren Schuppenschicht (Cuticula) zu gewährleisten und eine Penetration der Oxidationsfarbstoffvorprodukte in das Haar zu ermöglichen.

Erfindungsgemäße Verfahren sind dadurch gekennzeichnet, dass das kosmetische Mittel (M1) einen pH-Wert von pH 9,2 bis pH 10,5, aufweist. Die Einstellung dieser pH-Werte kann bevorzugt unter Verwendung der zuvor angeführten Alkalisierungsmittel erfolgen.

Um das natürliche und multitonale Färbeergebnis am Ende des erfindungsgemäßen Verfahrens besonders deutlich und überraschend hervortreten zu lassen, ist das Vorbehandlungsmittel (M1) für sich alleine vorzugsweise nicht in der Lage, als separates Bleich-, Aufhell- oder Färbemittel eingesetzt zu werden. Hierzu ist es von besonderem Vorteil, wenn die kosmetischen Mittel (M1) frei sind von Oxidationsmitteln, insbesondere frei sind von Wasserstoffperoxid und/oder Persulfaten.

In Verfahrensschritt c) des erfindungsgemäßen Verfahrens wird ein kosmetisches Mittel (M2) auf die keratinischen Fasern appliziert, welche noch mit dem Mittel (M1) beaufschlagt sind. Dieses kosmetische Mittel (M2), welches im nachfolgenden auch als Färbemittel bezeichnet wird, enthält mindestens ein Oxidationsfarbstoffvorprodukt (M2-1) und mindestens ein Oxidationsmittel (M2-2).

Bevorzugte kosmetische Mittel (M2) enthalten mindestens ein Oxidationsfarbstoffvorprodukt vom Entwicklerund/oder Kupplertyp. Entsprechende erfindungsgemäße Verfahren, bei welchen das kosmetische Mittel (M2) als Oxidationsfarbstoffvorprodukt (M2-1) ein oder mehrere Oxidationsfarbstoffvorprodukte vom Entwicklertyp enthält, sind erfindungsgemäß bevorzugt.

Geeignete und bevorzugte Oxidationsfarbstoffvorprodukte vom Entwicklertyp wurden bereits weiter oben detailliert beschrieben. Die entsprechenden Verbindungen können auch in den Färbemitteln (M2) eingesetzt werden. Es hat sich jedoch gezeigt, dass sich der Einsatz von bestimmten Oxidationsfarbstoffvorprodukten vom Entwicklertyp in bestimmten Mengen in den Färbemitteln (M2) gut eignet, um besonders lebendige, wasch-, reib-, schweiß- und UV-echte multitonalen Färbungen zu erzeugen.

Besonders bevorzugte erfindungsgemäße Verfahren sind dadurch gekennzeichnet, dass das kosmetische Mittel (M2) als Oxidationsfarbstoffvorprodukt vom Entwicklertyp ein oder mehrere Verbindungen aus der Gruppe von p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)-propyl]amin und/oder deren physiologisch verträglichen Salze in einer Gesamtmenge von 0,0005 bis 3,0 Gew.-%, vorzugsweise von 0,001 bis 2,75 Gew.-%, bevorzugt von 0,0025 bis 2,5 Gew.-%, insbesondere von 0,005 bis 2,0 Gew.-%, bezogen auf das Gesamtgewicht des kosmetischen Mittels (M2), enthält.

vorzugsweise von 0,001 bis 2,75 Gew.-%, bevorzugt von 0,0025 bis 2,5 Gew.-%, insbesondere von 0,005 bis 2,0 Gew.-%, bezogen auf das Gesamtgewicht des kosmetischen Mittels (M2), enthält.

Weitere besonders bevorzugte erfindungsgemäße Verfahren sind dadurch gekennzeichnet, dass das kosmetische Mittel (M2) als Oxidationsfarbstoffvorprodukt vom Entwicklertyp ein oder mehrere Verbindungen aus der Gruppe von Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-und Amino-3-methylphenol und/oder deren physiologisch verträglichen Salze in einer Gesamtmenge von 0,0005 bis 3,0 Gew.-%, vorzugsweise von 0,001 bis 2,75 Gew.-%, bevorzugt von 0,0025 bis 2,5 Gew.-%, insbesondere von 0,005 bis 2,0 Gew.-%, bezogen auf das Gesamtgewicht des kosmetischen Mittels (M2), enthält.

Weitere besonders bevorzugte erfindungsgemäße Verfahren sind dadurch gekennzeichnet, dass das kosmetische Mittel (M2) als Oxidationsfarbstoffvorprodukt vom Entwicklertyp ein oder mehrere Verbindungen aus der Gruppe von 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on und/oder deren physiologisch verträglichen Salze in einer Gesamtmenge von 0,1 bis 3,5 Gew.-%, vorzugsweise von 0,3 bis 2,8 Gew.-%, bevorzugt von 0,4 bis 2,1 Gew.-%, insbesondere von 0,5 bis 1,6 Gew.-%, bezogen auf das Gesamtgewicht des kosmetischen Mittels (M2), enthält.

Weitere besonders bevorzugte erfindungsgemäße Verfahren sind dadurch gekennzeichnet, dass das kosmetische Mittel (M2) als Oxidationsfarbstoffvorprodukte vom Entwicklertyp mindestens eine der folgenden Kombinationen enthält: p-Toluylendiamin/2-(2-Hydroxyethyl)-p-phenylendiamin; p-Toluylendiamin/2-Methoxymethyl-p-phenylendiamin; p-Toluylendiamin/N,N-Bis-(2-hydroxyethyl)-p-phenylendiamin; p-Toluylendiamin/2-Methoxymethyl-p-phenylendiamin; p-Toluylendiamin/N-(4-Amino-3-methylphenyl)-N-[3-(1H-imidazol-1-yl)propyl]amin; p-Toluylendiamin/Bis-(2-hydroxy-5-aminophenyl)-methan; p-Toluylendiamin/4-und Amino-3-methylphenol; p-Toluylendiamin/4,5-Diamino-1-(2-hydroxyethyl)pyrazol; p-Toluylendiamin/2,4,5,6-Tetraaminopyrimidin; 2-(2-Hydroxyethyl)-p-phenylendiamin /2-Methoxymethyl-p-phenylendiamin; 2-(2-Hydroxyethyl)-p-phenylendiamin /N,N-Bis-(2-hydroxyethyl)-p-phenylendiamin; 2-(2-Hydroxyethyl)-p-phenylendiamin /2-Methoxymethyl-p-phenylendiamin; 2-(2-Hydroxyethyl)-p-phenylendiamin/N-(4-Amino-3-methylphenyl)-N-[3-(1H-imidazol-1-yl)propyl]amin; 2-(2-Hydroxyethyl)-p-phenylendiamin /Bis-(2-hydroxy-5-aminophenyl)methan; 2-(2-Hydroxyethyl)-p-phenylendiamin/4-Amino-3-methylphenol; 2-(2-Hydroxyethyl)-p-phenylendiamin /4,5-Diamino-1-(2-hydroxyethyl)pyrazol; 2-(2-Hydroxyethyl)-p-phenylendiamin /2,4,5,6-Tetraaminopyrimidin; 2-Methoxymethyl-p-phenylendiamin/2-(2-Hydroxyethyl)-p-phenylendiamin; 2-Methoxymethyl-p-phenylendiamin /2-Methoxymethyl-p-phenylendiamin; 2-Methoxymethyl-p-phenylendiamin /N,N-Bis-(2-hydroxyethyl)-p-phenylendiamin; 2-Methoxymethyl-p-phenylendiamin /2-Methoxymethyl-p-phenylendiamin; 2-Methoxymethyl-p-phenylendiamin /N-(4-Amino-3-methylphenyl)-N-[3-(1H-imidazol-1-yl)propyl]amin; 2-Methoxymethyl-p-phenylendiamin /Bis-(2-hydroxy-5-aminophenyl)methan; 2-Methoxymethyl-p-phenylendiamin/4-Amino-3-methylphenol; 2-Methoxymethyl-p-phenylendiamin /4,5-Diamino-1-(2-hydroxyethyl)pyrazol; 2-Methoxymethyl-p-phenylendiamin /2,4,5,6-Tetraaminopyrimidin und/oder 4-und Amino-3-methylphenol/4,5-Diamino-1-(2-hydroxyethyl)pyrazol und/oder deren physiologisch verträglichen Salze.

Erfindungsgemäß bevorzugt enthält das kosmetische Mittel (M2) weiterhin ein oder mehrere Oxidationsfarbstoffvorprodukte vom Kupplertyp.

Erfindungsgemäß bevorzugt eingesetzte Kupplerkomponenten werden aus einer der folgenden Klassen ausgewählt: m-Aminophenol, o-Aminophenol, m-Diaminobenzol, o-Diaminobenzol und/oder deren Derivate; Naphthalinderivate mit mindestens einer Hydroxygruppe; Di- beziehungsweise Trihydroxybenzol; Pyridinderivate; Pyrimidinderivate; bestimmte Indol-Derivate und Indolin-Derivate; Pyrazolonderivate (beispielsweise 1-Phenyl-3-methylpyrazol-5-on); Morpholinderivate (beispielsweise 6-Hydroxybenzomorpholin oder 6-Aminobenzomorpholin); Chinoxalinderivate (beispielsweise 6-Methyl-1,2,3,4-tetrahydrochinoxalin), sowie Gemische aus zwei oder mehreren Verbindungen aus einer oder mehreren dieser Klassen.

Bevorzugte m-Aminophenol-Kupplerkomponenten werden ausgewählt aus mindestens einer Verbindung aus der Gruppe von 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen. Bevorzugte m-Diaminobenzol-Kupplerkomponente werden ausgewählt aus mindestens einer Verbindung aus der Gruppe von m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen. Bevorzugte o-Diaminobenzol-Kupplerkomponenten werden ausgewählt aus mindestens einer Verbindung aus der Gruppe von 3,4-Diaminobenzoesäure und 2,3-Diamino-1-methylbenzol und deren physiologisch verträglichen Salzen. Bevorzugte Naphthalinderivate mit mindestens einer Hydroxygruppe werden ausgewählt aus mindestens einer Verbindung der Gruppe 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Bevorzugte Di- beziehungsweise Trihydroxybenzole und deren Derivate werden ausgewählt aus mindestens einer Verbindung aus der Gruppe von Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Bevorzugte Pyridinderivate werden ausgewählt aus mindestens einer Verbindung der Gruppe 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und deren physiologisch verträglichen Salzen. Bevorzugte Pyrimidinderivate werden ausgewählt aus mindestens einer Verbindung aus der Gruppe von 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen. Bevorzugte Indolderivate werden ausgewählt aus mindestens einer Verbindung aus der Gruppe von 4-Hydroxyindol, 6-Hydroxyindol und 7-Hydroxyindol und deren physiologisch verträglichen Salzen. Bevorzugte Indolinderivate werden ausgewählt aus mindestens einer Verbindung aus der von Gruppe 4-Hydroxyindolin, 6-Hydroxyindolin und 7-Hydroxyindolin und deren physiologisch verträglichen Salzen.

Erfindungsgemäß besonders bevorzugt verwendete Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salze. Ganz besonders bevorzugt sind Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie deren physiologisch verträglichen Salze.

Die Kupplerkomponenten sind in dem kosmetischen Mittel (M2) bevorzugt in einer Gesamtmenge von 0,0001 bis 2,0 Gew.-%, insbesondere von 0,001 bis 1,25 Gew.-%, bezogen auf das Gesamtgewicht des kosmetischen Mittels (M2), enthalten.

Der Einsatz von bestimmten Oxidationsfarbstoffvorprodukten vom Kupplertyp in bestimmten Mengen in den Färbemitteln (M2) resultiert in multitonalen Färbungen, welche besonders lebendige, wasch-, reib-, schweiß- und UV-echte Farben aufweisen.

Erfindungsgemäß bevorzugte Verfahren sind daher dadurch gekennzeichnet, dass das kosmetische Mittel (M2) als Oxidationsfarbstoffvorprodukt vom Kupplertyp ein oder mehrere Verbindungen aus der Gruppe von 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol und/oder deren physiologisch verträglichen Salze in einer Gesamtmenge von 0,001 bis 2,0 Gew.-%, vorzugsweise von 0,0025 bis 1,75 Gew.-%, bevorzugt von 0,0025 bis 1,5 Gew.-%, insbesondere von 0,005 bis 1,25 Gew.-%, bezogen auf das Gesamtgewicht des kosmetischen Mittels (M2), enthält.

Weitere erfindungsgemäß bevorzugte Verfahren sind dadurch gekennzeichnet, dass das kosmetische Mittel (M2) als Oxidationsfarbstoffvorprodukt vom Kupplertyp ein oder mehrere Verbindungen aus der Gruppe von 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol und/oder deren physiologisch verträglichen Salze in einer Gesamtmenge von 0,001 bis 2,0 Gew.-%, vorzugsweise von 0,0025 bis 1,75 Gew.-%, bevorzugt von 0,0025 bis 1,5 Gew.-%, insbesondere von 0,005 bis 1,25 Gew.-%, bezogen auf das Gesamtgewicht des kosmetischen Mittels (M2), enthält.

Weitere erfindungsgemäß bevorzugte Verfahren sind dadurch gekennzeichnet, dass das kosmetische Mittel (M2) als Oxidationsfarbstoffvorprodukt vom Kupplertyp ein oder mehrere Verbindungen aus der Gruppe von Resorcin, 2-Methylresorcin und/oder 4-Chlorresorcin in einer Gesamtmenge von 0,001 bis 2,0 Gew.-%, vorzugsweise von 0,0025 bis 1,75 Gew.-%, bevorzugt von 0,0025 bis 1,5 Gew.-%, insbesondere von 0,005 bis 1,25 Gew.-%, bezogen auf das Gesamtgewicht des kosmetischen Mittels (M2), enthält.

Weitere erfindungsgemäß bevorzugte Verfahren sind dadurch gekennzeichnet, dass das kosmetische Mittel (M2) als Oxidationsfarbstoffvorprodukt vom Kupplertyp ein oder mehrere Verbindungen aus der Gruppe von 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on und/oder deren physiologisch verträglichen Salze in einer Gesamtmenge von 0,001 bis 2,0 Gew.-%, vorzugsweise von 0,0025 bis 1,75 Gew.-%, bevorzugt von 0,0025 bis 1,5 Gew.-%, insbesondere von 0,005 bis 1,25 Gew.-%, bezogen auf das Gesamtgewicht des kosmetischen Mittels (M2), enthält.

Weitere erfindungsgemäß bevorzugte Verfahren sind dadurch gekennzeichnet, dass das kosmetische Mittel (M2) als Oxidationsfarbstoffvorprodukt vom Kupplertyp mindestens eine der folgenden Kombinationen enthält: Resorcin/3-Aminophenol; 2-Methylresorcin/3-Aminophenol; 4-Chlorresorcin/3-Aminophenol; Resorcin/5-Amino-2-methylphenol; 2-Methylresorcin/5-Amino-2-methylphenol; 4-Chlorresorcin/5-Amino-2-methylphenol; Resorcin/2-Hydroxy-4-aminophenoxyethanol; 2-Methylresorcin/2-Hydroxy-4-aminophenoxyethanol; 4-Chlorresorcin/2-Hydroxy-4-aminophenoxyethanol; Resorcin/2-Amino-3-hydroxypyridin; 2-Methylresorcin/2-Amino-3-hydroxypyridine; 4-Chlorresorcin/2-Amino-3-hydroxypyridine; Resorcin/3-Amino-2-methylamino-6-methoxypyridin; 2-Methylresorcin/3-Amino-2-methylamino-6-methoxypyridin; 4-Chlorresorcin/3-Amino-2-methylamino-6-methoxypyridin; Resorcin/2,6-Dihydroxy-3,4-dimethylpyridin; 2-Methylresorcin/2,6-Dihydroxy-3,4-dimethylpyridin und/oder 4-Chlorresorcin/2,6-Dihydroxy-3,4-dimethylpyridin und/oder deren physiologisch verträglichen Salze.

Vorzugsweise enthält das Vorbehandlungsmittel (M1) die Oxidationsfarbstoffvorprodukte vom Entwicklertyp (M1-1) in einer höheren Gesamtstoffmenge als das kosmetische Mittel (M2) die Oxidationsfarbstoffvorprodukte (M2-1). Dies führt zu besonders intensiven und lebendigen multitonalen Färbungen, welche darüber hinaus eine hohe Beständigkeit gegen Umwelteinflüsse, wie beispielsweise Haarwäschen, Schweiß, UV-Licht oder Reibung, aufweisen.

Gemäß einer besonders bevorzugten Ausführungsform der vorliegenden Erfindung weist das Stoffmengenverhältnis der Gesamtstoffmenge aller Oxidationsfarbstoffvorprodukte vom Entwicklertyp (M1-1) in dem kosmetischen Mittel (M1) zur Gesamtstoffmenge aller Oxidationsfarbstoffvorprodukte (M2-1) in dem kosmetischen Mittel (M2) einen Wert (M1-1)/(M2-1) von 1: 5 bis 1 : 2, vorzugsweise von 1 : 1 bis 2 : 1, bevorzugt von 80 : 1 bis 120 : 1, weiter bevorzugt von 180 : 1 bis 250 : 1, insbesondere von 400 : 1 bis 600 :1, auf. Unter Gesamtstoffmenge wird hierbei die Summe der Stoffmengen aller in dem kosmetischen Mittel (M1) eingesetzten Oxidationsfarbstoffprodukte vom Entwicklertyp (M1-1) bzw. die Summe der Stoffmengen aller in dem kosmetischen Mittel (M2) enthaltenen Oxidationsfarbstoffvorprodukte (M2-1) verstanden.

Eine Variation der Nuancierung der multitonalen Färbung ist durch entsprechende Wahl der in den kosmetischen Mitteln (M1) und (M2) eingesetzten Oxidationsfarbstoffvorprodukte möglich. Für eine sehr natürlich wirkende multitonale Färbung mit weichen Übergängen sind erfindungsgemäße Verfahren bevorzugt, bei welchen die kosmetischen Mittel (M1) und (M2) identische Oxidationsfarbstoffvorprodukte vom Entwicklertyp und vom Kupplertyp enthalten.

Werden stärkere Kontraste gewünscht, welche sich in einem brillanteren multitonalen Erscheinungsbild der Färbung äußern, haben sich erfindungsgemäße Verfahren bewährt, bei welchen die kosmetischen Mittel (M1) und (M2) unterschiedliche Oxidationsfarbstoffvorprodukte vom Entwicklertyp enthalten.

Im Rahmen des erfindungsgemäßen Verfahrens kann es vorgesehen sein, dass die kosmetischen Mittel (M1) und (M2) identische Inhaltsstoffe enthalten. Bevorzugt unterscheiden sich die kosmetischen Mittel (M1) und (M2) jedoch in mindestens einem Inhaltsstoff voneinander.

Das Färbemittel (M2) kann zusätzlich direktziehende Farbstoffe enthalten, um eine ausgewogene Nuancierung der multitonalen Färbungen zu gewährleisten. Gemäß einer bevorzugten Ausführungsform des Erfindungsgegenstands enthält das kosmetische Mittel (M2) zusätzlich mindestens einen direktziehenden Farbstoff aus der Gruppe von anionischen direktziehenden Farbstoffen, kationischen direktziehenden Farbstoffen, nichtionischen direktziehenden Farbstoffen sowie deren Mischungen.

Geeignete und bevorzugte direktziehende Farbstoffe wurden bereits im Zusammenhang mit dem Vorbehandlungsmittel (M1) detailliert beschrieben. Die entsprechenden Verbindungen können auch in den Färbemitteln (M2) eingesetzt werden. Es hat sich jedoch gezeigt, dass sich der Einsatz von bestimmten direktziehenden Farbstoffen in bestimmten Mengen in den Färbemitteln (M2) besonders gut eignet, da in diesem Fall eine besonders ausgewogene Nuancierung der multitonalen Färbungen gewährleistet werden kann.

Im Rahmen einer Ausführungsform des erfindungsgemäßen Verfahrens besonders bevorzugt eingesetzte kosmetische Mittel (M2) enthalten daher zusätzlich mindestens einen direktziehenden Farbstoff, welcher ausgewählt ist aus der Gruppe von 2-Amino-6-chloro-4-nitrophenol, HC Blue 12, HC Yellow 2, HC Violet 14D und/oder deren physiologisch verträglichen Salzen sowie deren Mischungen.

Erfindungsgemäße Verfahren im Rahmen dieser Ausführungsform sind dadurch gekennzeichnet, dass das kosmetische Mittel (M2) den mindestens einen direktziehenden Farbstoff in einer Gesamtmenge von 0,00001 bis 5,0 Gew.-%, vorzugsweise von 0,000025 bis 4,0 Gew.-%, bevorzugt von 0,00005 bis 3,0 Gew.-%, weiter bevorzugt von 0,0001 bis 2,0 Gew.-%, insbesondere von 0,0005 bis 1,5 Gew.-%, bezogen auf das Gesamtgewicht des kosmetischen Mittels (M2), enthält.

Die Färbemittel (M2) können weiterhin zusätzliche Wirk-, Hilfs- und Zusatzstoffe enthalten, um die Färbeleistung zu verbessern und weitere gewünschte Eigenschaften der kosmetischen Mittel (M2) einzustellen.

Erfindungsgemäß bevorzugt enthält das kosmetische Mittel (M2) daher zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Tensiden; (iv) Alkalisierungsmitteln; sowie (v) deren Mischungen.

Es hat sich als vorteilhaft erwiesen, wenn die kosmetischen Mittel (M2) ebenfalls mindestens ein Verdickungsmittel enthalten. Bezüglich dieser Verdickungsmittel bestehen keine prinzipiellen Einschränkungen. Geeignete Verdickungsmittel sind die im Zusammenhang mit dem Vorbehandlungsmittel (M1) angeführten Verbindungen, welche zur Verdickung der Färbemittel (M2) ebenfalls eingesetzt werden können. Daneben können auch die nachfolgend angeführten organischen und anorganischen Verdickungsmittel verwendet werden.

Geeignete Verdickungsmittel sind anionische, synthetische Polymere; kationische, synthetische Polymere; natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen; nichtionische, synthetische Polymere, wie Polyvinylalkohol oder Polyvinylpyrrolidon; sowie anorganische Verdickungsmittel, insbesondere Schichtsilikate wie beispielsweise Bentonit, besonders Smektite, wie Montmorillonit oder Hectorit.

Die in dem erfindungsgemäßen Verfahren eingesetzten kosmetischen Mittel (M2) können als Verdickungsmittel auch zwitterionische Polymere enthalten, welche ausgewählt sind aus der Gruppe der

- Copolymere aus Dimethyl-diallylammoniumsalzen und Acrylsäure, z.B. Polyquaternium-22,
- Copolymere aus Dimethyl-diallylammoniumsalzen und Methacrylsäure,
- Copolymere aus N,N,N-Trimethyl-3-[(2-methyl-1-oxo-2-propen-1-yl)amino]-1-propanaminiumsalzen und Acrylsäure,
- Copolymere aus N,N,N-Trimethyl-3-[(2-methyl-1-oxo-2-propen-1-yl)amino]-1-propanaminiumsalzen und Methacrylsäure,
- Copolymere aus N,N,N-Trimethyl-2-[(2-methyl-1-oxo-2-propen-1-yl)amino]-1-ethanaminiumsalzen und Acrylsäure,
- Copolymere aus N,N,N-Trimethyl-2-[(2-methyl-1-oxo-2-propen-1-yl)amino]-1-ethanaminiumsalzen und Methacrylsäure,
- Copolymere aus N,N,N-Trimethyl-3-[(2-methyl-1-oxo-2-propen-1-yl)amino]-1-propanaminiumsalzen, Acrylsäure und Acrylamid, z.B. Polyquaternium-53
- Copolymere aus N,N,N-Trimethyl-3-[(2-methyl-1-oxo-2-propen-1-yl)amino]-1-propanaminiumsalzen, Methacrylsäure und Acrylamid,
- Copolymere aus 1-Ethenyl-3-methyl-1H-imidazoliumsalzen, 1-Ethenyl-1H-imidazol, 1-Ethenyl-2-pyrrolidinon und Methacrylsäure, z.B. Polyquaternium-86.

Auch Gemische der vorgenannten zwitterionischen Polymere können zur Verdickung der kosmetischen Mittel (M2) eingesetzt werden.

Die in dem erfindungsgemäßen Verfahren eingesetzten Färbemittel (M2) können ebenfalls die im Zusammenhang mit dem Vorbehandlungsmittel (M1) beschriebenen Alkohole aus der Gruppe von Arachylalkohol (Eisocan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), Heneicosylalkohol (Heneicosan-1-ol), Behenylalkohol (Docosan-1-ol), Erucylalkohol ((13Z)-Docos-13-en-1-ol) und Brassidylalkohol ((13E)-Docosen-1-ol) in einer Gesamtmenge von 0,1 bis 10,0 Gew.-%, vorzugsweise von 1,4 bis 8,5 Gew.-%, bevorzugt von 1,8 bis 8,0 Gew.-%, insbesondere von 2,0 bis 7,0 Gew.-%, bezogen auf das Gesamtgewicht des kosmetischen Mittels (M2), enthalten.

Vorzugsweise werden die Färbemittel (M2) als flüssige Zubereitung bereitgestellt und diesen Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt. Die in den Färbemitteln (M2) einsetzbaren Tensiden und Emulgatoren sind bereits im Zusammenhang mit den Vorbehandlungsmitteln (M1) beschrieben worden.

Im Rahmen dieser Ausführungsform ist es bevorzugt, wenn die anionischen Tenside in den kosmetischen Mitteln (M2) in einer Gesamtmenge von 0,1 bis 45 Gew.%, vorzugsweise von 1 bis 30 Gew.%, insbesondere von 1 bis 15 Gew.%, bezogen auf die Gesamtmenge des kosmetischen Mittels (M2), eingesetzt werden.

Darüber hinaus ist es im Rahmen dieser Ausführungsform bevorzugt, wenn die nichtionischen und/oder zwitterionischen und/oder amphoteren Tenside in einer Gesamtmenge von 0,1 bis 45 Gew.%, vorzugsweise von 1 bis 30 Gew.%, insbesondere von 1 bis 15 Gew.%, bezogen auf die Gesamtmenge des kosmetischen Mittels (M2), eingesetzt werden.

Auch das kosmetische Mittel (M2) kann mindestens ein Alkalisierungsmittel enthalten. Geeignete Alkalisierungsmittel sowie deren einsetzbare Gesamtmengen sind bereits im Zusammenhang mit dem Vorbehandlungsmittel (M1) angeführt worden. Die Einstellung eines basischen pH-Werts unter Verwendung des mindestens einen Alkalisierungsmittels ist erforderlich, um eine Öffnung der äußeren Schuppenschicht (Cuticula) zu gewährleisten und eine Penetration der Oxidationsfarbstoffvorprodukte in das Haar zu ermöglichen.

Erfindungsgemäße Verfahren sind dadurch gekennzeichnet, dass das kosmetische Mittel (M2) einen pH-Wert von pH 9,2 bis pH 10,5, aufweist.

Um eine lebendige multitonale Färbung zu erzielen, ist es von Vorteil, wenn es bei dem sequentiellen Auftragen der kosmetischen Mittel (M1) und (M2) nicht zu starken pH-Wertschwankungen kommt, da hierdurch eine nur unzureichende Penetration der keratinischen Fasern und damit auch ein verschlechtertes Färbeergebnis auftreten können. Erfindungsgemäße Verfahren sind daher dadurch gekennzeichnet, dass das kosmetischen Mittel (M1) und das kosmetischen Mittel (M2) identische pH-Werte aufweisen.

Die Oxidationsfarbstoffvorprodukte (Entwickler und Kuppler) selbst sind nicht gefärbt. Die Bildung der eigentlichen Farbstoffe erfolgt erst im Verlauf der Anwendung durch den Kontakt der Oxidationsfarbstoffvorprodukte mit einem Oxidationsmittel (vorzugsweise Wasserstoffperoxid). In einer chemischer Reaktion werden die als Oxidationsfarbstoffvorprodukte eingesetzten Entwickler (wie beispielsweise p-Phenylendiamin-Derivate oder p-Aminophenolderivate) durch Wasserstoffperoxid zunächst oxidativ in eine reaktive Zwischenstufe, auch Chinonimin oder Chinondiimin genannt, überführt, welche dann in einer oxidativen Kupplungsreaktion mit den Kupplern zum jeweiligen Farbstoff reagiert.

Die kosmetischen Mittel (M2) enthalten daher zusätzlich ein oder mehrere Oxidationsmittel (M2-2). Als Oxidationsmittel kommen Persulfate, Peroxodisulfate, Chlorite, Hypochlorite und insbesondere Wasserstoffperoxid und/oder eines seiner festen Anlagerungsprodukte an organische oder anorganische Verbindungen in Frage.

Erfindungsgemäß bevorzugte Verfahren sind daher dadurch gekennzeichnet, dass das kosmetische Mittel (M2) mindestens ein Oxidationsmittel (M2-2) aus der Gruppe von Persulfaten, Peroxodisulfaten, Chloriten, Hypochloriten, Wasserstoffperoxid sowie dessen feste Anlagerungsprodukte an Harnstoff, Melamin, Polyvinylpyrrolidon und Natriumborat, bevorzugt Wasserstoffperoxid, in einer Gesamtmenge von 0,5 bis 10 Gew.-%, vorzugsweise von 1 bis 10 Gew.-%, insbesondere von 2 bis 10 Gew.-%, bezogen auf das Gesamtgewicht des kosmetischen Mittels (M2), enthält. Falls als Oxidationsmittel Wasserstoffperoxid sowie dessen feste Anlagerungsprodukte eingesetzt werden, berechnet sich die vorstehend genannte Gesamtmenge auf 100%iges $H_2O_2$.

In einer weiteren bevorzugten Ausführungsform ist das kosmetische Mittel (M2) ein Mittel zum Färben und gegebenenfalls gleichzeitigen Aufhellen keratinischer Fasern, welches Wasserstoffperoxid in einer Gesamtmenge von 0,5 bis 15 Gew.-%, vorzugsweise 1 bis 12,5 Gew.-%, bevorzugt 1,5 bis 10 Gew.-%, insbesondere 1,5 bis 6,5 Gew.-%, bezogen auf das Gesamtgewicht des kosmetischen Mittels (M2), enthält. Die vorstehend genannte Gesamtmenge an Wasserstoffperoxid ist hierbei auf 100%iges $H_2O_2$ bezogen.

Zur Erzielung einer verstärkten Aufhell- und Bleichwirkung kann das kosmetische Mittel (M2) weiterhin mindestens ein Peroxo-Salz enthalten. Geeignete Peroxo-Salze sind anorganische Peroxoverbindungen.

Zur Erzielung einer verstärkten Aufhell- und Bleichwirkung kann das kosmetische Mittel (M2) weiterhin mindestens ein Peroxo-Salz enthalten. Geeignete Peroxo-Salze sind anorganische Peroxoverbindungen, bevorzugt ausgewählt aus der Gruppe von Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden sowie deren Mischungen. Besonders bevorzugt sind Peroxodisulfate, insbesondere Ammoniumperoxodisulfat, Kaliumperoxodisulfat und Natriumperoxodisulfat.

Die zuvor genannten Peroxosalze sind in einer Gesamtmenge von 0,5 bis 20 Gew.-%, vorzugsweise 1 bis 12,5 Gew.-%, bevorzugt 2,5 bis 10 Gew.-%, insbesondere 3 bis 6 Gew.-%, bezogen auf das Gesamtgewicht des kosmetischen Mittels (M2), enthalten.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht. Üblicherweise werden oxidative Färbemittel daher in Form eines aus zwei Komponenten bestehenden "Kits" (Mehrkomponenten-Verpackungseinheit) angeboten, wobei die erste Komponente die Oxidationsfarbstoffvorprodukte und gegebenenfalls direktziehenden Farbstoffe sowie ein Alkalisierungsmittel (beispielsweise Ammoniak) und die zweite Komponente das Oxidationsmittel enthält.

In einer weiteren Ausführungsform der vorliegenden Erfindung sind daher kosmetische Mittel (M2) bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden und wobei ein Container ein Färbemittel (M2a), welches in einem kosmetischen Träger mindestens ein Oxidationsfarbstoffvorprodukt enthält, und ein weiterer Container eine Oxidationsmittelzubereitung (M2b), enthaltend mindestens ein Oxidationsmittel, enthält.

Das Färbemittel (M2a) enthält hierbei bevorzugt die zuvor im Zusammenhang mit dem kosmetischen Mittel (M2) aufgeführten Oxidationsfarbstoffvorprodukte vom Entwicklertyp und/oder Kupplertyp, gegebenenfalls mindestens einen direktziehenden Farbstoff und gegebenenfalls mindestens einen zuvor im Zusammenhang mit dem kosmetischen Mittel (M2) aufgeführten Wirk-, Hilfs- oder Zusatzstoff. Die Oxidationsmittelzubereitung (M2b) enthält bevorzugt ein Oxidationsmittel in Form von Wasserstoffperoxid und/oder eines seiner festen Anlagerungsprodukte an organische oder anorganische Verbindungen, wie Harnstoff, Melamin sowie Natriumborat.

Solche Oxidationsmittelzubereitungen (M2b) sind vorzugsweise wässrige, fließfähige Oxidationsmittelzubereitungen. Dabei sind bevorzugte Zubereitungen (M2b) dadurch gekennzeichnet, dass die fließfähige Oxidationsmittelzubereitung (M2b) 40 bis 90 Gew.-%, vorzugsweise 50 bis 90 Gew.-%, bevorzugt 55 bis 89 Gew.-%, weiter bevorzugt 60 bis 87 Gew.-%, insbesondere 65 bis 85 Gew.-%, bezogen auf das Gesamtgewicht der Oxidationsmittelzubereitung (M2b), Wasser enthält.

Bevorzugt beträgt die Gesamtmenge an Oxidationsmittel, insbesondere Wasserstoffperoxid, in der Oxidationsmittelzubereitung (M2b) 0,5 bis 12 Gew.-%, vorzugsweise 1,0 bis 10 Gew.-%, insbesondere 1,5 bis 6,0 Gew.-%, bezogen auf das Gesamtgewicht der Oxidationsmittelzubereitung (M2b). Falls als Oxidationsmittel Wasserstoffperoxid sowie dessen feste Anlagerungsprodukte eingesetzt werden, berechnet sich die vorstehend genannte Gesamtmenge auf 100%iges $H_2O_2$.

Erfindungsgemäß kann die Oxidationsmittelzubereitung (M2b) auch zusammen mit einem Katalysator auf das Haar aufgebracht werden, welcher die Oxidation der Farbstoffvorprodukte aktiviert. Solche Katalysatoren sind z. B. bestimmte Enzyme, Iodide, Chinone oder Metallionen.

Es hat sich als vorteilhaft erwiesen, wenn die Oxidationsmittelzubereitungen (M2b) zur Stabilisierung des Oxidationsmittels, insbesondere des Wasserstoffperoxids, zusätzlich mindestens einen Stabilisator oder Komplexbildner enthalten. Besonders bevorzugte Stabilisatoren sind insbesondere EDTA und EDDS sowie Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

## Patentansprüche

1. Verfahren zum oxidativen Färben von keratinischen Fasern, wobei das Verfahren die folgenden Verfahrensschritte in der angegebenen Reihenfolge umfasst:

   a) Applikation eines kosmetischen Mittels (M1) auf die keratinischen Fasern, wobei das kosmetische Mittel (M1) nur auf einzelne Strähnchen appliziert wird,
   b) Einwirkenlassen des Mittels (M1) für einen Zeitraum von 30 Sekunden bis 35 Minuten bei einer Temperatur von 30 °C bis 120 °C auf den keratinischen Fasern,
   c) Applikation eines kosmetischen Mittels (M2) auf die mit dem kosmetischen Mittel (M1) beaufschlagten keratinischen Fasern,
   d) Einwirkenlassen der kosmetischen Mittel (M1) und (M2) für einen Zeitraum von 1 bis 70 Minuten auf den keratinischen Fasern,
   e) Ausspülen der kosmetischen Mittel (M1) und (M2),

   **dadurch gekennzeichnet, dass**

   - das kosmetische Mittel (M1)
   mindestens ein Oxidationsfarbstoffvorprodukt vom Entwicklertyp enthält (M1-1),
   mindestens ein Oxidationsfarbstoffvorprodukt vom Kupplertyp enthält (M1-2),
   mindestens einen direktziehenden Farbstoff enthält (M1-3), und
   mindestens ein Tensid in einer Gesamtmenge von 4,0 bis 12 Gew.-%, bezogen auf das Gesamtgewicht des kosmetischen Mittels (M1), enthält (M1-4),
   einen pH-Wert von 9,2 bis 10,5 aufweist,

0 Gew.-%, bezogen auf das Gesamtgewicht des kosmetischen Mittels (M1), Oxidationsmittel enthält,
- das kosmetische Mittel (M2)
mindestens ein Oxidationsfarbstoffvorprodukt enthält (M2-1), und
mindestens ein Oxidationsmittel enthält (M2-2),
- das kosmetischen Mittel (M1) und das kosmetischen Mittel (M2) identische pH-Werte aufweisen.

2. Verfahren nach Anspruch 1, **dadurch gekennzeichnet, dass** das kosmetische Mittel (M1) in Verfahrensschritt b) für einen Zeitraum von 30 Sekunden bis 30 Minuten, bevorzugt von 30 Sekunden bis 20 Minuten, insbesondere von 30 Sekunden bis 15 Minuten, auf den keratinischen Fasern Einwirken gelassen wird.

3. Verfahren nach einem der vorangehenden Ansprüche, **dadurch gekennzeichnet, dass** die kosmetischen Mittel (M1) und (M2) in Verfahrensschritt d) für einen Zeitraum von 1 bis 60 Minuten, vorzugsweise von 5 bis 50 Minuten, insbesondere von 10 bis 45 Minuten, Einwirken gelassen werden.

4. Verfahren nach einem der vorangehenden Ansprüche, **dadurch gekennzeichnet, dass** das kosmetische Mittel (M1) das mindestens eine Oxidationsfarbstoffvorprodukt vom Entwicklertyp (M1-1) in einer Gesamtmenge von 0,0025 bis 10,0 Gew.-%, vorzugsweise von 0,004 bis 8,0 Gew.-%, bevorzugt 0,005 bis 5,0 Gew.-%, insbesondere 0,01 bis 3,5 Gew.-%, bezogen auf das Gesamtgewicht des kosmetischen Mittels (M1), enthält.

5. Verfahren nach einem der vorangehenden Ansprüche, **dadurch gekennzeichnet, dass** das kosmetische Mittel (M1) das mindestens eine Oxidationsfarbstoffvorprodukt vom Kupplertyp (M1-2) in einer Gesamtmenge von 0,001 bis 6,0 Gew.-%, vorzugsweise von 0,001 bis 5,5 Gew.-%, bevorzugt von 0,002 bis 4,5 Gew.-%, insbesondere von 0,005 bis 2,5 Gew.-%, bezogen auf das Gesamtgewicht des kosmetischen Mittels (M1), enthält.

6. Verfahren nach einem der vorangehenden Ansprüche, **dadurch gekennzeichnet, dass** das kosmetische Mittel (M1) den mindestens einen direktziehenden Farbstoff (M1-3) in einer Gesamtmenge von 0,0001 bis 6,0 Gew.-%, vorzugsweise von 0,0005 bis 5,5 Gew.-%, bevorzugt von 0,0008 bis 5,0 Gew.-%, weiter bevorzugt von 0,005 bis 4,5 Gew.-%, insbesondere von 0,001 bis 3,5 Gew.-%, bezogen auf das Gesamtgewicht des kosmetischen Mittels (M1), enthält.

7. Verfahren nach einem der vorangehenden Ansprüche, **dadurch gekennzeichnet, dass** das kosmetische Mittel (M1) eine dynamische Viskosität von 10 bis 300 mPa*s, vorzugsweise von 20 bis 200 mPa*s, bevorzugt von 30 bis 100 mPa*s, weiter bevorzugt von 40 bis 90 mPa*s, insbesondere von 50 bis 80 mPa*s, jeweils gemessen mit Brookfield RDV II+, Spindel Nr. 1, 100 rpm, 20 °C, aufweist.

8. Verfahren nach einem der vorangehenden Ansprüche, **dadurch gekennzeichnet, dass** das kosmetische Mittel (M2) als Oxidationsfarbstoffvorprodukt (M2-1) mindestens ein Oxidationsfarbstoffvorprodukt vom Entwickler- und/oder Kupplertyp enthält.

9. Verfahren nach einem der vorangehenden Ansprüche, **dadurch gekennzeichnet, dass** das Mengenverhältnis der Stoffmenge aller Oxidationsfarbstoffvorprodukte vom Entwicklertyp (M1-1) in dem kosmetischen Mittel (M1) zur Gesamtmenge aller Oxidationsfarbstoffvorprodukte (M2-1) in dem kosmetischen Mittel (M2) einen Wert (M1-1)/(M2-1) von 1: 5 bis 1 : 2, vorzugsweise von 1 : 1 bis 2 : 1, bevorzugt von 80 : 1 bis 120 : 1, weiter bevorzugt von 180 : 1 bis 250 : 1, insbesondere von 400 : 1 bis 600 :1, aufweist.

10. Verfahren nach einem der vorangehenden Ansprüche, **dadurch gekennzeichnet, dass** das kosmetische Mittel (M2) mindestens ein Oxidationsmittel (M2-2) aus der Gruppe von Persulfaten, Peroxodisulfaten, Chloriten, Hypochloriten, Wasserstoffperoxid sowie dessen feste Anlagerungsprodukte an Harnstoff, Melamin, Polyvinylpyrrolidon und Natriumborat, bevorzugt Wasserstoffperoxid, in einer Gesamtmenge von 0,5 bis 10 Gew.-%, vorzugsweise von 1 bis 10 Gew.-%, insbesondere von 2 bis 10 Gew.-%, bezogen auf das Gesamtgewicht des kosmetischen Mittels (M2), enthält.

**Claims**

1. A method for oxidatively dyeing keratin fibers, the method comprising the following method steps in the specified sequence:

a) applying a cosmetic agent (M1) to the keratin fibers, the cosmetic agent (M1) being applied only to individual strands,
b) allowing agent (M1) to act on the keratin fibers for a period of from 30 seconds to 35 minutes at a temperature of from 30 °C to 120 °C,
c) applying a cosmetic agent (M2) to the keratin fibers that have been subjected to cosmetic agent (M1),
d) allowing cosmetic agents (M1) and (M2) to act on the keratin fibers for a period of from 1 to 70 minutes,
e) rinsing off cosmetic agents (M1) and (M2),

**characterized in that**

- cosmetic agent (M1) contains
at least one developer-type oxidation dye precursor (M1-1),
at least one coupler-type oxidation dye precursor (M1-2),
at least one direct dye (M1-3), and
at least one surfactant (M1-4) in a total amount of from 4.0 to 12 wt.%, based on the total weight of cosmetic agent (M1),
has a pH of from 9.2 to 10.5,
contains 0 wt.%, based on the total weight of cosmetic agent (M1), oxidizing agent,
- cosmetic agent (M2) contains
at least one oxidation dye precursor (M2-1), and
at least one oxidizing agent (M2-2),
- cosmetic agent (M1) and cosmetic agent (M2) have identical pH values.

2. The method according to claim 1, **characterized in that**, in method step b), cosmetic agent (M1) is allowed to act on the keratin fibers for a period of from 30 seconds to 30 minutes, preferably from 30 seconds to 20 minutes, in particular from 30 seconds to 15 minutes.

3. The method according to one of the preceding claims, **characterized in that**, in method step d), cosmetic agents (M1) and (M2) are allowed to act for a period of from 1 to 60 minutes, preferably from 5 to 50 minutes, in particular from 10 to 45 minutes.

4. The method according to one of the preceding claims, **characterized in that** cosmetic agent (M1) contains the at least one developer-type oxidation dye precursor (M1-1) in a total amount of from 0.0025 to 10.0 wt.%, preferably from 0.004 to 8.0 wt.%, more preferably 0.005 to 5.0 wt.%, in particular 0.01 to 3.5 wt.%, based on the total weight of cosmetic agent (M1).

5. The method according to one of the preceding claims, **characterized in that** cosmetic agent (M1) contains the at least one coupler-type oxidation dye precursor (M1-2) in a total amount of from 0.001 to 6.0 wt.%, preferably from 0.001 to 5.5 wt.%, more preferably from 0.002 to 4.5 wt.%, in particular from 0.005 to 2.5 wt.%, based on the total weight of cosmetic agent (M1).

6. The method according to one of the preceding claims, **characterized in that** cosmetic agent (M1) contains the at least one direct dye (M1-3) in a total amount of from 0.0001 to 6.0 wt.%, preferably from 0.0005 to 5.5 wt.%, more preferably from 0.0008 to 5.0 wt.%, even more preferably from 0.005 to 4.5 wt.%, in particular from 0.001 to 3.5 wt.%, based on the total weight of cosmetic agent (M1).

7. The method according to one of the preceding claims, **characterized in that** cosmetic agent (M1) has a dynamic viscosity of from 10 to 300 mPa·s, preferably from 20 to 200 mPa·s, more preferably from 30 to 100 mPa·s, even more preferably from 40 to 90 mPa·s, in particular from 50 to 80 mPa·s, in each case measured using Brookfield RDV II+, spindle no. 1, 100 rpm, 20 °C.

8. The method according to one of the preceding claims, **characterized in that** cosmetic agent (M2) contains at least one developer-type and/or coupler-type oxidation dye precursor as the oxidation dye precursor (M2-1).

9. The method according to one of the preceding claims, **characterized in that** the ratio of the amount of substance of all the developer-type oxidation dye precursors (M1-1) in cosmetic agent (M1) to the total amount of all the oxidation dye precursors (M2-1) in cosmetic agent (M2) has a value (M1-1)/(M2-1) of from 1:5 to 1:2, preferably from 1:1 to 2:1, more preferably from 80:1 to 120:1, even more preferably from 180:1 to 250:1, in particular from 400:1 to 600:1.

10. The method according to one of the preceding claims, **characterized in that** cosmetic agent (M2) contains at least one oxidizing agent (M2-2) from the group of persulfates, peroxydisulfates, chlorites, hypochlorites, hydrogen peroxide and the solid addition products thereof to urea, melamine, polyvinylpyrrolidone and sodium borate, preferably hydrogen peroxide, in a total amount of from 0.5 to 10 wt.%, preferably from 1 to 10 wt.%, in particular from 2 to 10 wt.%, based on the total weight of cosmetic agent (M2).

**Revendications**

1. Procédé de coloration par oxydation de fibres kératiniques, le procédé comprenant les étapes de procédé suivantes, dans l'ordre indiqué :

     a) l'application d'un agent cosmétique (M1) sur les fibres kératiniques, l'agent cosmétique (M1) n'étant appliqué que sur des mèches individuelles,
     b) le fait de laisser agir l'agent (M1) sur les fibres kératiniques pendant une durée de 30 secondes à 35 minutes à une température de 30 °C à 120 °C,
     c) l'application d'un agent cosmétique (M2) sur les fibres kératiniques imprégnées d'agent cosmétique (M1),
     d) le fait de laisser agir les agents cosmétiques (M1) et (M2) sur les fibres kératiniques pendant une durée de 1 à 70 minutes,
     e) le rinçage des agents cosmétiques (M1) et (M2),

     **caractérisé en ce que**

     - l'agent cosmétique (M1)
     contient au moins un précurseur de colorant d'oxydation du type développeur (M1-1),
     contient au moins un précurseur de colorant d'oxydation du type coupleur (M1-2), contient au moins un colorant direct (M1-3), et
     contient au moins un tensioactif en une quantité totale de 4,0 à 12 % en poids, par rapport au poids total de l'agent cosmétique (M1) (M1-4),
     présente une valeur de pH de 9,2 à 10,5,
     contient 0 % en poids d'agent oxydant, par rapport au poids total de l'agent cosmétique (M1),
     - l'agent cosmétique (M2)
     contient au moins un précurseur de colorant d'oxydation (M2-1) et
     contient au moins un agent oxydant (M2-2),
     - l'agent cosmétique (M1) et l'agent cosmétique (M2) présentent des valeurs de pH identiques.

2. Procédé selon la revendication 1, **caractérisé en ce qu'**on laisse agir l'agent cosmétique (M1) sur les fibres kératiniques dans l'étape de procédé b) pendant une durée de 30 secondes à 30 minutes, de manière préférée de 30 secondes à 20 minutes, en particulier de 30 secondes à 15 minutes.

3. Procédé selon l'une des revendications précédentes, **caractérisé en ce qu'**on laisse agir les agents cosmétiques (M1) et (M2) dans l'étape de procédé d) pendant une durée de 1 à 60 minutes, de préférence de 5 à 50 minutes, en particulier de 10 à 45 minutes.

4. Procédé selon l'une des revendications précédentes, **caractérisé en ce que** l'agent cosmétique (M1) contient l'au moins un précurseur de colorant d'oxydation du type développeur (M1-1) en une quantité totale de 0,0025 à 10,0 % en poids, de préférence de 0,004 à 8,0 % en poids, de manière préférée de 0,005 à 5,0 % en poids, en particulier de 0,01 à 3,5 % en poids, par rapport au poids total de l'agent cosmétique (M1).

5. Procédé selon l'une des revendications précédentes, **caractérisé en ce que** l'agent cosmétique (M1) contient l'au moins un précurseur de colorant d'oxydation du type coupleur (M1-2) en une quantité totale de 0,001 à 6,0 % en poids, de préférence de 0,001 à 5,5 % en poids, de manière préférée de 0,002 à 4,5 % en poids, en particulier de 0,005 à 2,5 % en poids, par rapport au poids total de l'agent cosmétique (M1).

6. Procédé selon l'une des revendications précédentes, **caractérisé en ce que** l'agent cosmétique (M1) contient l'au moins un colorant direct (M1-3) en une quantité totale de 0,0001 à 6,0 % en poids, de préférence de 0,0005 à 5,5 % en poids, de manière préférée de 0,0008 à 5,0 % en poids, de manière davantage préférée de 0,005 à 4,5 % en poids, en particulier de 0,001 à 3,5 % en poids, par rapport au poids total de l'agent cosmétique (M1).

7. Procédé selon l'une des revendications précédentes, **caractérisé en ce que** l'agent cosmétique (M1) présente une viscosité dynamique de 10 à 300 mPa*s, de préférence de 20 à 200 mPa*s, de manière préférée de 30 à 100 mPa*s, de manière davantage préférée de 40 à 90 mPa*s, en particulier de 50 à 80 mPa*s, respectivement mesurée au moyen d'un viscosimètre Brookfield RDV II+, avec la broche n° 1 à 100 tr/min et 20 °C.

8. Procédé selon l'une des revendications précédentes, **caractérisé en ce que** l'agent cosmétique (M2) contient, en tant que précurseur de colorant d'oxydation (M2-1), au moins un précurseur de colorant d'oxydation du type développeur et/ou coupleur.

9. Procédé selon l'une des revendications précédentes, **caractérisé en ce que** le rapport quantitatif entre la quantité molaire de tous les précurseurs de colorants d'oxydation du type développeur (M1-1) dans l'agent cosmétique (M1) et la quantité totale de tous les précurseurs de colorants d'oxydation (M2-1) dans l'agent cosmétique (M2) présente une valeur (M1-1)/(M2-1) de 1:5 à 1:2, de préférence de 1:1 à 2:1, de manière préférée de 80:1 à 120:1, de manière davantage préférée de 180:1 à 250:1, en particulier de 400:1 à 600:1.

10. Procédé selon l'une des revendications précédentes, **caractérisé en ce que** l'agent cosmétique (M2) contient au moins un agent oxydant (M2-2) du groupe constitué des persulfates, des peroxodisulfates, des chlorites, des hypochlorites, du peroxyde d'hydrogène ainsi que de ses produits d'addition solides sur l'urée, de la mélamine, de la polyvinylpyrrolidone et du borate de sodium, de manière préférée du peroxyde d'hydrogène, en une quantité totale de 0,5 à 10 % en poids, de préférence de 1 à 10 % en poids, en particulier de 2 à 10 % en poids, par rapport au poids total de l'agent cosmétique (M2).

**IN DER BESCHREIBUNG AUFGEFÜHRTE DOKUMENTE**

**In der Beschreibung aufgeführte Patentdokumente**

- DE 201000140 U1
- DE 2913755 A1
- WO 03068177 A2
- DE 3025992
- WO 0115662 A1